# EUROPEAN PATENT APPLICATION

(11) **EP 3 123 931 A1**
(43) Date of publication of application: **01.02.2017**
(21) Application number: 15178802.3
(22) Date of filing: 29.07.2015
(51) Int. Cl.: A61B 5/00, A47G 9/10, A61F 5/56

(54) **HEAD SUPPORT FOR STOPPING AIRWAY DISORDERS**

(71) Applicant: Nitetronic Holding Limited, Grand Cayman KY1- 1112 (KY)
(72) Inventor: Von Janecek, Hubertus, 201206 Shanghai (CN); Herrnsdorf, Johannes, 58313 Herdecke (DE)
(74) Representative: Banse & Steglich

(57) **Abstract**

The invention relates to an airways disorder alleviating system (1) for stopping an ongoing and/or upcoming airways disorder of a sleeping individual, comprising:
- a resting surface (3) on which the individual at least partly is to rest;
- a head support (2) with an active layer (4) being configured to selectively adapt the height of the head support (2) section by section;
- at least one spatial sensor (7) with at least one detection means (71) arranged in or on at least one of the resting surface (3) and the head support (2) for contactlessly detecting at least one of a position of a body part and a biomotion of a body part in a sensing range (R, R'); and
- a control unit (6) configured to actuate the active layer (4) for selectively adapting the height of the head support (2) depending on the at least one the detected position and the detected biomotion so that the head of the individual is moved.

## Description

### Technical field

The present invention relates to head supports, in particular pillows. Furthermore, the present invention relates to detecting airway disorders during sleep and measures for suppressing and/or stopping an irregular breathing event. Particularly, the invention relates to measures for detecting breathing activities of an individual who rests on the head support. Furthermore, the present invention relates to detecting a head position on a head support.

### Related art

A head support for stopping snoring of a sleeping individual is known by document US 2014/0310878, wherein the head support has a head resting surface for the head of a sleeping individual to rest on. The head support has an active layer with an arrangement of neighboring deforming elements for controlling the height of the head support section by section. To detect a position of the head resting on the head resting surface a sensing layer is provided which is arranged between the head resting surface and the active layer.

So far, the position of the head on the head support is detected by using a sensing layer or an array of sensors which are configured to detect a change of an electrical quantity depending on a change of pressure applied on the head support by the weight of the head. By the arrangement of the one or more sensors exposed to the weight of the head the position of the head can be determined. The permanent impact and movement of the head on the head support may result in a continuous mechanical stress on the sensing layer which may lead to a failure.

During sleep of an individual, pathologic effects such as snoring and/or sleep apnea can reduce oxygen absorption which may be dangerous to health of the sleeping individual. It has been found that moving head or body of the sleeping individual during snoring or during an ongoing sleep apnea event may immediately resolve the snoring or sleep apnea event, respectively, and urges the individual to continue normal breathing.

From prior art, various approaches are known to detect irregularities in respiration of a sleeping individual due to sleep apnea and trying to actively move the body and/or the head of the sleeping individual .

In document WO 2014/114438 A1, a sleeping pillow for alleviating a sleep apnea is disclosed. The sleeping pillow has an adjusting means system which can be actuated for changing the position of a head resting on the sleeping pillow by way of a control device and which has sensor means for detecting the position of the head. The control device is configured so that in addition sensor means signals from further sensor means for detecting respiratory activities of a person can be detected. In order to detect a sleep apnea of a person using the sleeping pillow, the further sensor means comprise sensors for detecting a breathing frequency or breathing movements of the chest of the person or sensors for detecting the nose dynamic pressure of a person. The adjusting system means systems can be actuated with respect to the sensor means signals of the further sensor means to alleviate or to avoid a sleep apnea event.

From document DE 10 2009 039 915 A1, an apparatus for the detection and signaling of the sleep-relating breathing dysfunction of an individual is known. Therein, it is described that a sensor is fixated at the head of an individual so that mechanical inertia at the head of the individual can be measured and analyzed to obtain head alignment information, breathing information about a breathing behavior and snoring information about the snoring behavior of the individual.

Document CN 103 222 909 A discloses a pillow with air chambers. Between the surfaces, a sensor is provided which is connected to a processor. The processor is configured to collect a sleep respiratory rate of a user. The sensor is configured as a sound sensor for acquiring the number of breaths, breath frequency rate and other sleep information from the user.

Document CN 102 743 246 A discloses an inflatable pillow with air chambers for changing the position of a head of an individual resting on the pillow. When a sleep apnea occurs, the individual's head is moved by locally actuating the air chambers of the pillow. Detection of the sleep apnea may be performed by means of a wrist assembly for detecting the patient's pulse and blood oxygen concentration, means for detecting a sleeping position or by means of a belly assembly for detecting a swing and abdomen abdominal breathing gas flow component.

Document CN1557270 A discloses a pillow with inflatable air chambers which are controlled depending on an input of a respiratory sensor signal.

Document KR100711701 B1 discloses a pillow for preventing a sleep-related breathing disorder. The pillow has a number of chambers in which a pressure can be regulated. By detecting the characteristics of pressure in each of the chambers, it can be estimated whether a breathing disorder is caused by comparing the pressure of each chamber with control data indicating an optimal pressure pattern data. The pressure of the air chambers is regulated, if a breathing disorder is detected.

The approaches discussed above try to determine a sleep apnea by analyzing a sound or pressure signal or a body movement detected by external or body mounted sensors, which renders the whole system complex, unreliable and/or inconvenient to use.

It is therefore an object of the present invention to provide an improved head support which may have robust position detection of a head of a sleeping individual. Furthermore, it is an object to provide an improved head support which has a robust detection of an airway disorder during sleep of an individual. Furthermore, it is an object to reliably detect of an occurrence of an airway disorder event and moving a head of a sleeping individual depending on the detection of a sleep apnea event.

### Summary of the invention

This object has been achieved by the airways disorder alleviating system according to claim 1 and the head support and the method according to the further independent claims.

Further embodiments are indicated in the depending claims.

According to a first aspect, an airways disorder alleviating system for stopping an ongoing and/or upcoming airways disorder of a sleeping individual is provided , comprising:
- a resting surface on which the individual at least partly is to rest;
- a head support with an active layer being configured to selectively adapt the height of the head support section by section;
- at least one spatial sensor with at least one detection means arranged in or on at least one of the resting surface and the head support for detecting at least one of a position of a body part and a biomotion of a body part in a sensing range in an contactless manner; and
- a control unit configured to actuate the active layer for selectively adapting the height of the head support depending on the at least one detected position and detected biomotion so that the head of the individual is moved.

One idea of the above airways disorder alleviating system is the use of a spatial sensor to provide a detection sensor signal by sensing, in a contactless manner, at least one of the presence and the position of a body part such as a head of the individual and a minor movement of an internal boundary and/or of an outer surface of the body part of the individual resting on the head support. A minor movement of the internal boundary and/or of the outer surface of the body part is referred herein to as a biomotion for sake of simplicity.

The spatial sensor is configured to span a detection range by establishing a field, such as at least one of an electromagnetic field, a sonic field and/or an electrical field. A presence and/or any changes of positions of any body parts thereof within the detection range results in a distortion or any kind of measurable influence on the field which can be detected and analysed. In case of emitted electromagnetic energy or sonic energy electromagnetic or sonic energy is transmitted and reflected portions of the electromagnetic or sonic energy can be respectively received and detected. By analyzing reflection characteristic the presence and/or the position of the body part and/or biomotion in or of the body part can be determined.

In case of an electrical field the field is distorted by the presence and/or the position of a body part and/or the biomotion of the body part which can be detected and analyzed. Particularly, capacitive effects of the impact of the presence and/or the position of a body part and/or the biomotion of the body part can be detected, measured and/or analyzed.

The spatial sensor may be configured to detect a position of a body part of the individual, such as the head on the head support and to actuate selected ones of deformation elements in the head support depending on the detected position of the body part so as to move the head of the individual resting on the resting surface.

The spatial sensor may be configured to also detect a biomotion, which is provided as the detection sensor signal of the spatial sensor as well as the position of a body part or an external or inner boundary on or in the body part above the resting surface.

It has been determined that a detected biomotion of a sleeping individual often has a low frequency portion which strongly relates to its respiratory (breathing) activity. Furthermore, other portions of the detection sensor signal can be related to the heart-rate, blood pressure and other vibrations in the individual's body caused by body functions.

Analyzing and/or filtering the detection sensor signal therefore relates in a more or less periodic signal which indicates a respiratory activity, i.e. the breath-in/breath-out movements of the individual or the head position on the head support.

The arrangement of the spatial sensor in, on or under the resting area of the sleeping individual, i.e. beneath the sleeping individual, such as in the interior of the head support, allows to keep the distance between the spatial sensor and the individual's body short and substantially constant which may lead to a good accuracy and a very accurate contactless detection with respect to the detection sensor signals. This high accuracy is particularly beneficial for reliably detecting detection sensor signals with signal portions from which indications of an upcoming and/or ongoing airways disorder event such as a snoring or a sleep apnea can be derived alone or in combination with indications of blood-oxygen saturation (SpO2), a heart-rate/heart-beat, body movement pattern, vibrations, sounds, including snoring.

Furthermore, the arrangement of the spatial sensor in, on or under the resting area of the sleeping individual allows to focus the detection range towards the head or the body of an individual resting on the head support, i.e. in an upward direction for detection of at least one of a position of the head and of a biomotion of or in the head, and/or in a direction at least partly extending along a body axis of the individual resting on the head support. A possible detection range may be directed inclined to the upward direction into a direction of the body axis of the individual.

Thereby, a detection of a biomotion of body parts of another individual nearby can be excluded from detection so that the detection of the detection sensor signal is less disturbed. This allows a more reliable detection of a respiratory change stopping of the breathing activities of the specific individual resting on the head support. In contrast to other techniques for the detection of respiratory activity, such as analyzing of a sound signal based on a detection of a breathing sound or a contact-bound motion detection by e.g. chest belt or the like, the above head support allows for a more accurate acquisition of a breathing activity by excluding distortion caused by other movements of non-head body parts of the individual. Furthermore, it is possible to implement the above head support without the need of external means for the detection of the respiratory activities.

It may be provided that the at least one spatial sensor further comprises a signal extraction means for receiving a detection sensor signal from the detection means and for extracting at least one of a position sensor signal indicating the position of the body part of the sleeping individual and a biomotion sensor signal indicating periodic body characteristics of the sleeping individual from the at least one detection sensor signal.

The control unit may be configured to detect an upcoming and/or ongoing airways disorder, particularly a sleep apnea event, depending on the at least one biomotion sensor signal indicating as a body characteristics a respiratory activity of the sleeping individual.

Additionally or alternatively, the body characteristics may include at least one of a blood-oxygen saturation, a heart-rate, a body movement pattern, vibrations, and a sound emitted by the individual, wherein the control unit is configured to determine an upcoming and/or ongoing airways disorder, such as a snoring or sleep apnea event if at least one of the body characteristics is paused for a predetermined time and a predetermined body characteristics pattern is detected.

Furthermore, the detections means may be configured to have a sensing range which is directed one of upward perpendicular to the resting surface and upward inclined in a plane defined by the direction perpendicular to the resting surface and by a longitudinal direction of the resting surface.

Furthermore, the control unit may be configured to select one or more of deformation elements of the active layer to be actuated depending on the position sensor signal.

According to an embodiment, the at least one detection means of the at least one spatial sensor may be arranged at one of in and on the resting surface and is displaced from the head support along a longitudinal direction of the resting surface.

Moreover, the detection means of the at least one spatial sensor may be arranged at least one of on and in the interior of the head support.

Furthermore, the signal extraction means of the at least one spatial sensor may be arranged in one of the head support and the control unit.

It may be provided that the at least one detection means includes an antenna, wherein the at least one spatial sensor is configured to emit radio frequency energy via an antenna as the at least one detection means and to provide the at least one detection sensor signal indicating the respiratory activity using a Doppler effect.

Additionally or alternatively, the at least one detection means may include a sonic transducer, wherein the at least one spatial sensor is configured to emit sonic, particularly ultrasonic energy and to provide the at least one detection sensor signal, wherein the signal extraction means is configured to analyze the detection sensor signal using an acoustic Doppler effect.

Additionally or alternatively, the at least one detection means may include an electrode arrangement having a number of electrodes, wherein the detection means is configured to generate an electrical field in the sensing range, wherein the at least one spatial sensor is configured to detect a distortion of the electrical field due to at least one of a position of a body part of the sleeping individual and a biomotion of a body part of the sleeping individual and to provide at least one detection sensor signal indicating at least one of the position of the body part and the respiratory activity.

Additionally or alternatively, the detection means may include a capacitive detection means, wherein the at least one detection means includes an electrode arrangement having a number of electrodes which are particularly neighbored and substantially arranged along a direction, wherein the signal extraction means is configured for measuring at least one capacity between at least two of the electrodes and for providing at least one detection sensor signal depending on the at least one measured capacity so that at least one of a position sensor signal indicating the position of the body part of the sleeping individual and a biomotion sensor signal indicating periodic body characteristics of the sleeping individual is extractable from the at least one detection sensor signal.

By detecting an interruption of the so detected respiratory activity for a predetermined time period, such as 5 seconds, an ongoing sleep apnea can be determined and a head movement initiated. Furthermore, by permanently analyzing the respiratory activity and comparing it with a provided or previously stored or extracted respiratory pattern a potentially upcoming sleep apnea can be detected.

According to a further aspect, an airways disorder alleviating system for stopping an ongoing and/or upcoming airways disorder of a sleeping individual is provided, comprising:
- a resting surface on which the individual at least partly is to rest;
- a head support with an active layer being configured to selectively adapt the height of the head support section by section;
- at least one spatial sensor with at least one detection means arranged in or on at least one of the resting surface and the head support for detecting a position of a body part in a sensing range in a contactless manner; and
- a control unit configured to actuate the active layer for selectively adapting the height of the head support depending on the detected position so that the head of the individual is moved.

According to a further aspect, an airways disorder alleviating system for stopping an ongoing and/or upcoming airways disorder of a sleeping individual is provided, comprising:
- a resting surface on which the individual at least partly is to rest;
- a head support with an active layer being configured to selectively adapt the height of the head support section by section;
- at least one spatial sensor with at least one detection means arranged in or on at least one of the resting surface and the head support for detecting, in a contactless manner, a biomotion of a body part in a sensing range; and
- a control unit configured to actuate the active layer for selectively adapting the height of the head support depending on the detected biomotion so that the head of the individual is moved.

According to a further aspect a head support for stopping an ongoing and/or upcoming airways disorder of a sleeping individual is provided, comprising:
- an active layer being configured to selectively adapt the height of the head support in sections;
- at least one detection means of at least one spatial sensor arranged on an upper surface of the head support or in the interior of the head support for contactlessly detecting at least one of a position of a body part and a biomotion of a body part in a sensing range; and
- a control unit configured to actuate the active layer for selectively adapting the height of the head support in sections depending on the at least one detected position and detected biomotion so that the head of the individual is moved.

It may be provided that the detection means is configured to have the sensing range directed one of upward perpendicular to the upper surface and upward inclined in a plane defined by the direction perpendicular to the upper surface and by a longitudinal direction of the resting surface.

Moreover, the at least one detection means may include one of:
- an antenna, wherein the at least one spatial sensor is configured to emit radio frequency energy via the antenna and to provide the at least one detection sensor signal indicating the respiratory activity using a Doppler effect; and
- a capacitive proximity detection means with an electrode arrangement having a number of electrodes, wherein the detection means is configured to generate an electrical field in the sensing range, wherein the at least one spatial sensor is configured to detect an distortion of the electrical field due to at least one of a position and a biomotion of a body part of the sleeping individual and to provide the at least one detection sensor signal.

Moreover, the electrode arrangement may be arranged one of below or above the deformation elements, wherein particularly the electrode arrangement has two electrode layers wherein at least one of the electrode layers is formed on the deformation elements, particularly as a coating.

According to a further aspect a head support for stopping an ongoing and/or upcoming airways disorder of a sleeping individual is provided, comprising:
- an active layer being configured to selectively adapt the height of the head support in sections;
- at least one detection means of at least one spatial sensor arranged on an upper surface of the head support or in the interior of the head support for contactlessly detecting a position of a body part in a sensing range; and
- a control unit configured to actuate the active layer for selectively adapting the height of the head support in sections depending on the detected position so that the head of the individual is moved.

According to a further aspect a head support for stopping an ongoing and/or upcoming airways disorder of a sleeping individual is provided, comprising:
- an active layer being configured to selectively adapt the height of the head support in sections;
- at least one detection means of at least one spatial sensor arranged on an upper surface of the head support or in the interior of the head support for detecting a biomotion of a body part in a sensing range in a contactless manner; and
- a control unit configured to actuate the active layer for selectively adapting the height of the head support in sections depending on the detected biomotion so that the head of the individual is moved.

According to a further aspect a method for stopping an ongoing and/or upcoming airways disorder of a sleeping individual is provided, comprising the steps of:
- contactlessly detecting at least one of a position and a biomotion in a sensing range, particularly perpendicular to a resting surface on which the head of the individual is to rest, and to provide at least one detection sensor signal; and
- from the detection sensor signal, extracting at least one of a position sensor signal indicating a position of a head of the sleeping individual and a biomotion sensor signal indicating a periodic body characteristics of the sleeping individual;
- actuating at least one selected of the deformation elements depending on the at least one position sensor signal and the detection sensor signal so that a head of the individual resting on the resting surface is moved.

### Brief description of the drawings

Embodiments of the present invention will be described with reference to the accompanying drawings in which:
- Figure 1: schematically shows a system including a head support and an underlay;
- Figure 2: is more detailed view of the head support and the control unit;
- Figure 3: shows a cross-sectional view through the system of Figure 1 indicating detection ranges;
- Figure 4: is an exploded view of a head support with an internal spatial sensor;
- Figure 5: is more detailed illustration of a configuration of a spatial sensor; and
- Figure 6: is an exploded view of a further head support with an internal spatial sensor;
- Figure 7: is an exploded view of a further head support with an internal spatial sensor.

### Description of embodiments

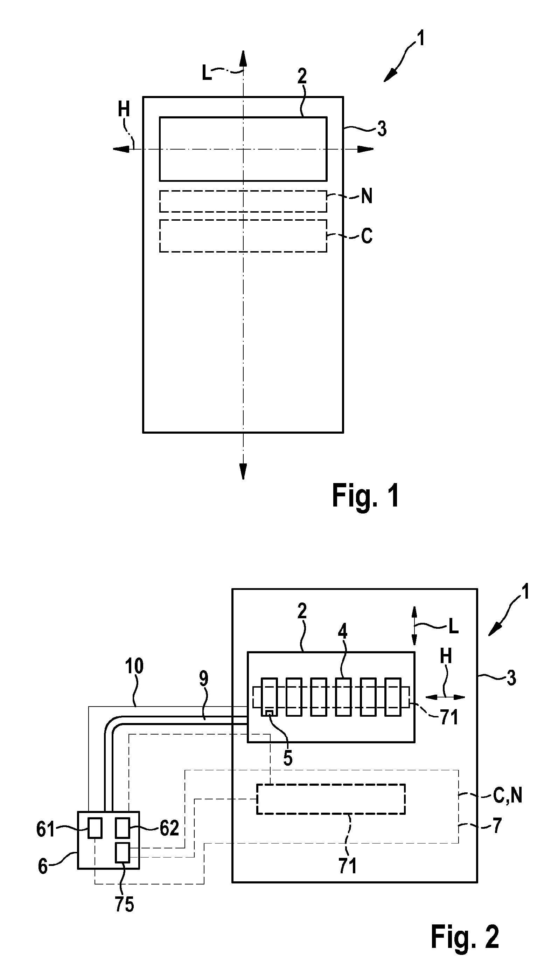

Figure 1 schematically shows a system 1 including a head support 2, such as a pillow, and an underlay 3 as a resting surface such as a mattress of a bed which corresponds to a resting surface. The system 1 serves for an individual to rest on.

The underlay 3 has a lateral direction H and a longitudinal direction L so that the individual lying thereon with its longitudinal body axis along the longitudinal direction L of the underlay 3. Substantially, the head support 2 can be freely located on the underlay 3 but it is preferred to arrange the head support 2 at or close to one end of the underlay 3 with respect to the longitudinal direction L like it is common for placing a pillow on a bed. The longitudinal L and lateral directions H of the head support 2 then substantially correspond to the longitudinal and lateral directions L, H of the underlay 3.

As shown in Figure 2 in more detail, an airways disorder alleviating system 1 is schematically shown including the head support 2 on the underlay 3. The head support 2 can be made in the form of a pillow or the like. The head support 2 may comprise a number of deformation elements 41 such as inflatable air chambers, electrically driven height actuators or any kind of actuators which may change its height to deform an upper surface of the head support 2 in an adaptable manner, wherein the upper surface defines a substantial plane or slightly convex surface of the head support 2. The deformation elements 41 can be arranged closely neighbored or (e.g. equally) distanced to each other along the lateral direction H or as an array along a lateral and longitudinal direction H, L of the head support 2 or along any two directions different thereto. So the deformation elements 41 may form an active layer 4 capable of deforming (changing height of) the upper surface of the head support 2 in sections (i.e. section by section). To keep the deformation elements 41 aligned a common carrier for the deformation elements 41 can be provided, wherein the deformation elements 41 are attached to the carrier.

Each of the deformation elements 41 is configured to adapt its height with respect to a direction perpendicular to the longitudinal and lateral directions L, H depending on control signals, so that the contour of the upper surface of the head support 2 can be adapted in sections (section by section).

In case the deformation elements 41 are formed as air chambers, each air chamber can be associated with a valve 5 (exemplarily shown for just one deformation element, but provided in each deformation element 41) to inflate or deflate. Each of the air chambers may be connected with an air pump 61 (or pressured air reservoir) preferably located in a control unit 6. The control unit 6 can be located externally of the head support 2 or in the interior thereof. An air tube 9 connects to each of the air chambers 3 via a respective valve 5 which can be controlled by control means 62 of the control unit 6. The control means 62 are connected via at least one control line 10 which can be along the air tube 9 between the control unit 6 and the head support 2. Control signals are provided to the valves 5 of the head support 2 via the control line 10.

By inflating, i.e. filling an air chamber with pressured air or increasing the pressure in the air chamber, or deflating, i.e. removing air from the air chamber or reducing the air pressure in the air chamber, the height of the head support can be adapted sectionwise so that the surface of the head support 2 on which a head of the individual is resting on, can be deformed so that the head is moved due to gravity, i.e. tilted along a longitudinal and/or lateral directions L, H.

As commonly know the deformation elements 41 can be controlled depending on a detection of snoring which can be detected by at least one sound sensor such as a microphone or by at least one pressure sensor at the deformation elements 41 (in case they are formed as air chambers).

For the position detection of a head of a sleeping individual on the head support 2 a position detector may be provided. The position detector may be configured as a sensing layer for detecting the position where the weight of a head lying on the head support 2 impacts the upper surface of the head support 2. Such a sensing layer needs to have a resolution to identify the deformation element 41 upon which the head of the individual is resting on the head support 2. This allows to selectively actuate the specific deformation element(s) 41 which is/are appropriate to have the head of the individual moved.

Alternatively or additionally to the snoring related control, a spatial sensor 7 is provided. In other embodiments, more than one spatial sensor 7 can be provided. The spatial sensor 7 has a signal extraction means 75 and one or more detection means 71. The detection means 71 may preferably be formed as a radio frequency transceiver (flat or rod antenna), a sonic transducer or as a capacitive proximity detecting means.

If the detection means 71 is formed as a radio frequency transceiver a single antenna, multiple antennas or an antenna array may be comprised.

In case the detection means 71 is formed as a capacitive proximity detecting means an electrode arrangement for establishing an electrical (near-)field is comprised. By measuring a capacity of the electrodes which depends on distances between the electrode arrangement and a head or body part or an outer surface or inner boundary thereof a position of the head or other body part or a biomotion can be derived. The capacitive proximity detecting means uses a capacitive sensing method to detect the change of capacitances with respect to the electrodes of the spatial sensor 7 due to the presence of a head or body part of an individual in a proximity thereof. This is based on that any conductive objects or object with high permittivity such as human skin, which is located nearby the capacitive proximity detecting means, can impact the electrode capacitance.

The detection means 71 can be internal of the head support 2 to be under the head of the individual. Optionally, the detection means 71 may be arranged external from the head support 2 in or on the resting surface laterally displaced from the head support 2 along the longitudinal direction L to be under a neck or a chest (see Figure 1) of the individual resting on the underlay 3. A neck portion N and a chest portion C are displaced from the position of the head support 2 along the longitudinal direction and indicate the positions above which the individual's neck and head, respectively, are located when resting on the underlay 3 with its head on the head support 2.

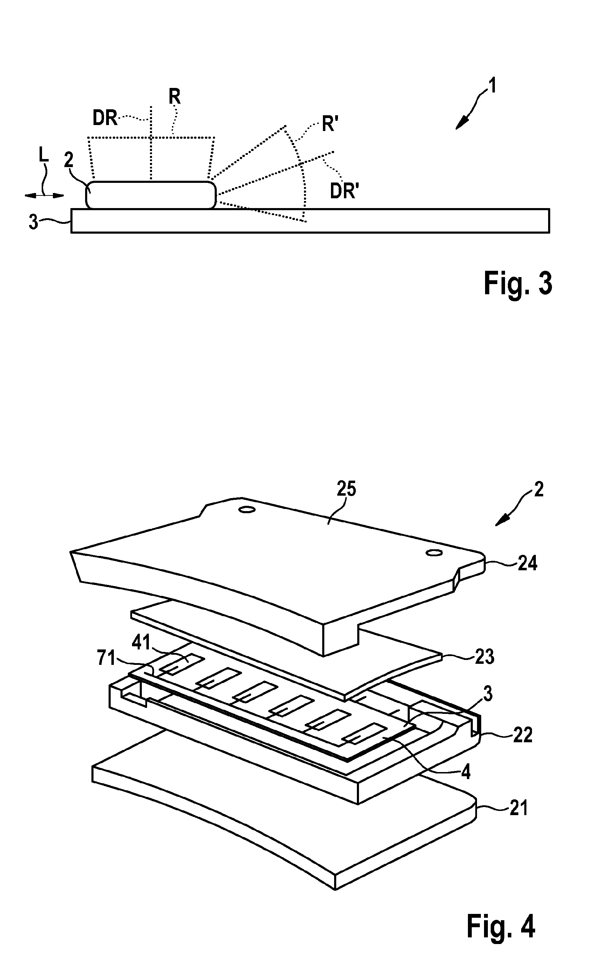

As can be seen in a cross sectional view of Figure 3, the detection means 71 of the spatial sensor 7 has a sensing range R whose main sensing direction DR may be substantially perpendicular both to the lateral L and to the longitudinal direction H of the head support 2 or underlay 3, respectively, i.e. into a (upward) direction to the upper surface of the head support 2 or in case of an external detection means 71 into a direction to an upper surface of the underlay 3. Additionally or alternatively, if the detection means 71 of the spatial sensor 7 is arranged in the head support 2, a further main sensing direction DR' of a further sensing range R' may be substantially directed from the head support 2 at least partly into a longitudinal direction L towards the chest of the individual resting on the underlay 3. Particularly, the further main sensing direction DR' can, as shown in Figure 3, be directed inclined between the longitudinal direction L and the (upward) direction perpendicular to the lateral L and to the longitudinal direction H.

The signal extraction means 75 may be located at the place of the detection means 71 or remote therefrom. The signal extraction means 75 is configured to extract and analyze the detection sensor signals and to provide position sensor signals and/or biomotion sensor signals.

The biomotion sensor signals are related to a biomotion of one or more external (skin) or internal boundaries in the head or body of the individual such as the motion of skin or the motion of trachea walls, vocal chords or the like. The biomotion of the one or more internal boundaries may be related to a respiratory activity of the individual so that the one or more biomotion sensor signals may have characteristics that include a relation to the respiratory action. The control means 62 of the control unit 6 is configured to receive the biomotion sensor signal from the signal extraction means 75 and to detect an airways disorder such as a sleep apnea event or a snoring, by analyzing them.

The control means 62 of the control unit 6 may be further configured to suitably compensate temperature influences on the position sensor signal and/or the biomotion sensor signal.

In case the detection means 71 is incorporated in the head support 2 or has a fixed arrangement with respect to the head support 2, the position sensor signals can be used to determine the position of a head on the upper surface of the head support 2. As the resolution of the above techniques applied by the detection means 71 of the spatial sensor 7 is high it provides a resolution which is appropriate to identify the one or more deformation elements 41 upon which the head of the individual is resting on the head support 2. This allows to selectively drive/actuate specific one or more of the deformation elements 41 which is/are appropriate to have the head of the individual moved in a predetermined manner.

As described above, the spatial sensor 7 may be formed as any kind of contactless sensor at least the detection means 71 of which is located at (in, on or under) the resting surface for the individual's body. The detection means 71 is configured/placed to have a sensing range R which is determined by the main sensing direction DR directing from the resting surface upward so that any micro-movements in the body of the individual can be detected. In case the detection means 71 is located in the head support 2 additionally the position of the head of the sleeping individual can be detected.

Moreover, the spatial sensor 7 may be formed as any kind of contactless sensor at least the detection means 71 of which is located in the head support 2. The detection means 71 is configured/placed to have a sensing range R' which is determined by the main sensing direction DR' directing from the head support 2 oblique in a plane defined by the longitudinal direction L and the (upward) direction perpendicular to the lateral L and to the longitudinal direction When on the underlay3, the main sensing direction DR' is directed from the head support 2 inclined toward a foot end of the underlay 3.

Preferably, the signal extraction means 75 may be arranged within the control unit 6. In this embodiment, the detection means 71 is connected by wires 11 or wireless with signal extraction means 75 in the control unit 6 either directly or via (e.g. fed-through) the head support 2 as illustrated by the dashed line.

In the following embodiments for head supports 2 are described which can be used in an airways disorder alleviating system 1 as e.g. shown in Figure 2.

Figure 4 shows an exploded view of a head support 2 with a detection means 71 of a spatial sensor 7 as a radio frequency transceiver. In case the detection means 71 is formed as a single radio frequency flat comb antenna it may be arranged above the active layer 4 of the deformation elements 41 of the head support 2. In other embodiments the detection means 71 may be arranged under the active layer 4. The active layer 4 of the head support 2 corresponds to the means by which the height of the head support 2 can be adapted section by section by actuating, e.g. by inflating or deflating, the deformation elements 41.

Substantially, the antenna(s) may have different configurations and can be formed as a rod, comb V- or flat antenna, arrangement of antennas, antenna arrays or the like and can be arranged on any portion of the head support 2 as long as detection of movements of the head on the head support 2 is not affected.

If the detection means 71 has a sensing range R substantially directed in the upward direction, both the position of the head of the sleeping individual resting on the upper surface of the head support 2 and a biomotion of an external and inner boundary of the head, neck or chest of the sleeping individual can be detected. Particularly, the biomotion of an external or inner boundary of the head of the sleeping individual can be detected independent of the position of the head resting on the head support 2.

The head support 2 comprises a number of layers which has a base layer 21 providing a rigid base support and the active layer 4 for positioning the deformation elements 41 and a frame means 22 for keeping it in a predefined position. The detection means 71 may be arranged above (as shown) or below the deforming elements 4 and substantially extends above them in at least one lateral direction thereof. On top of the detection means 71 and the active layer 4, a buffer layer 23 for (damping) flattening the surface contour and a cover layer 24 providing the upper surface 25 on which the head of the individual resting thereon rests.

In further embodiments as indicated in Figure 1 (dashed lines) it is provided to arrange a head support 2 (without the detection means 71) on the underlay 3 wherein the detection means 71 of the spatial sensor 7 is arranged external of the head support 2 on the surface of the underlay 3 or in the underlay 3. The detection means 71 of the spatial sensor 7 may be arranged on the underlay 3 directly under the head support 2 or displaced from the head support 2 along a longitudinal direction L of the underlay 3. When the detection means 71 is arranged displaced from the head support 2, it may be located beneath a neck portion or chest portion of an individual resting on the underlay 3 with its head resting on the head support 2 so that movements of internal boundaries of the individual's body can be detected.

In one embodiment, the spatial sensor 7 may include a radio frequency antenna as a detection means 71 which is operated to function as a radio frequency Doppler sensor. The radio frequency antenna can be used to transmit a radio frequency energy within a frequency range of 10 MHz to 100 GHz from the radio frequency antenna upward and to receive/detect the energy of a respective reflected received signal to construct the detection sensor signal by the signal extraction means 75.

The technique for detection of a biomotion, i.e. a motion of an internal boundary in the head or body of the individual, is explained in document US 2014/0163343 A1 which is incorporated herein by reference and it is therefore not described in detail.

Furthermore, the detection means 71 of the spatial sensor 7 can include an ultrasonic transducer to emit and direct an ultrasonic signal in an upward direction with respect to the resting surface of the underlay 3 and/or head support 2 and to detect reflected portions of the ultrasonic signals. The ultrasonic transducer can be located above or under the active layer 4. Due to the acoustic Doppler effect motions of internal boundaries due to respiratory activities of the individual can be detected and a respirator characteristic (breathing characteristic) can be derived thereof.

The ultrasonic transducer of the spatial sensor 7 can be located on a specific position particularly in a center position of the active layer 4 and have a fan-shaped or cone-shaped emission and detection coverage. Reflected energy of the ultrasonic signal can be received by the transducer and by analyzing the propagation delay motion of external and internal boundaries can be detected and extracted from the received ultrasonic signal to obtain detection sensor signals to be forwarded to the control means 62.

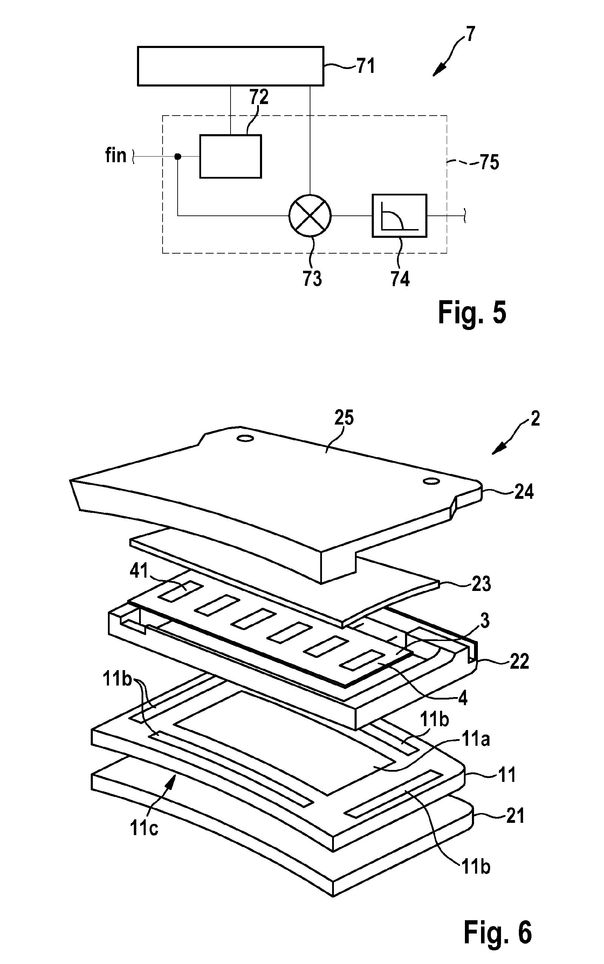

In Figure 5, components of a radio frequency spatial sensor 7 are indicated. The components may comprise the signal extraction means 75 and be integrated together with the antenna (detection means 71) into the head support 2. Alternatively the signal extraction means 75 may be a separate unit and be integrated in the control unit 6 or in the head support 2.

The radio frequency spatial sensor 7 has a transceiving antenna as the detection means 71 which is connected to a radio frequency amplifier 72 which is supplied with an input radio frequency signal which may be a pulsed or shaped radio frequency signal of a radio frequency between 10 MHz to 100 GHz. Furthermore, the antenna as well as the input radio signal are connected with a mixer 73 to detect a resulting phase shifted modulated signal indicative for the biomotion to be detected. The modulated signal is then low-pass filtered in a filter 74 to obtain the detection sensor signal to be further analyzed for a detection of a position of a head of the individual or of an airways disorder such as a snoring or a sleep apnea event.

The control means 62 is configured to drive the deformation elements 41 in case an ongoing and/or upcoming airways disorder event is detected. The deformation elements 41 are driven so that a head resting on the head support 4 will start being moved thereby preventing the sleep apnea event to occur or causing the sleeping individual to start breathing again.

An ongoing sleep apnea event will be determined by monitoring the detection sensor signal which has respiratory characteristics of a low frequency of 0,5Hz to 2Hz during normal respiration. If the detection sensor signal lacks the periodic characteristics of the low frequency for a predetermined time such as 3 -10 seconds then a sleep apnea event may be concluded and the actuation of the head support may be initiated.

Furthermore, the spatial sensor 7 can be configured to extract more than one detection sensor signal whose origins are located at different boundaries. The detection sensor signals can be correlated to extract the biomotion related to the respiratory activity.

Furthermore, biomotion of an internal boundary can be caused by other body functions such as heart-beat, emission of sound by vocal cords and the like. The accord of the biomotions are detected and reflected in the detection sensor signal. By applying suitable filters 74 a heart-rate signal (signal portion in a range between 50 and 150 Hz) and a sound signal emitted by the individual (signal portion in a range above 150 Hz) can be detected.

An upcoming sleep apnea event can be determined by monitoring the detection sensor signal which has characteristics of a low frequency of 0,5Hz to 2Hz during normal respiration. The low frequency portion of the detection sensor signal is compared with a predetermined respiratory pattern and in case of a resemblance between the low frequency portion of the detection sensor signal and the predetermined respiratory pattern an upcoming sleep apnea event may be detected.

To improve the detection of an upcoming sleep apnea event the characteristics of other frequency portions of the detection sensor signal can be considered. In this case a predetermined biomotion pattern can be defined in multiple frequency ranges to be compared with respective frequency ranges to the characteristics of the detected detection sensor signal. In case of resemblance or identity an upcoming sleep apnea event can be detected.

Figure 6 shows an exploded view of a further head support 2 with a capacitive proximity detecting means as a detection means 71 of a spatial sensor 7. Substantially, the arrangement corresponds to the embodiment to Figure 4.

The capacitive proximity detecting means is part of a 3D sensor technology which utilizes an electric field for proximity sensing. By means of an electrode arrangement of electrodes an electrical field can be generated. The electrical field may have any frequency of between 10 kHz and 1 GHz. As the wavelength is much larger than the dimensions of the electrodes 91 the magnetic component is practically zero and a quasi-static electrical near-field is established.

The near-field is established in a perpendicular direction above the electrode arrangement. If a head of the sleeping individual intrudes the electrical field, the field becomes distorted. If the head of the sleeping individual moves in the electrical field, the distortion of the field changes. By using an electrode arrangement 11 of the capacitive proximity detection means the position of the head can be clearly detected. This technique is well known in the art e.g. from Microchip "GestIC© design guide", 2013-2015 Microchip Technology Inc, ISBN: 978-1-63276-973-2 and is incorporated herein by reference.

The electrode arrangement 11 of the capacitive proximity detecting means may have a plurality of electrodes forming a substantially plane array having a first layer with a rectangular inner electrode 11 a and four side electrodes 11 b and a second layer with a common electrode 11c. The second layer with the common electrode 11 c is arranged in a plane opposite to the upper surface of the head support 2 with respect to the first layer. Each of the side electrodes 11 b extends along a side of the inner electrode 11a. The extension of the common electrode 11c in the second layer substantially fully covers the inner 11a and side electrodes 11 b of the first layer and is located under the first layer, i.e. opposite to the sensing range R, R' with respect to the first layer. The common electrode 11c serves as the source of the electrical field while the inner 11a and side electrodes 11 b detect capacity changes with respect to the head positioned or moving in the electrical field. Various different arrangements of electrodes 11a, 11 b are possible. The electrode arrangement 11 may include one or more of such electrode arrays.

The electrode arrangement 11 may be located below or above the active layer 4 as long as the distance between the head and the electrode arrangement 11 is within a sensing R, R' range of the electrical field detecting means. Furthermore, the electrodes may be formed as a patterned conductive coating on the deformation elements 41 and/or the active layer 4 formed thereof.

The signal extraction means 75 may include a circuitry which may extract a position information of the head based on a measured indication of the distortion of the electrical field. This may e.g. be performed by capacity measurements between the head and each of the electrodes.

Furthermore, the electrical field detecting means may detect periodic variations of the resulting sensor signal which may relate to conductivity changes of the skin of the sleeping individual and or breathing activities due to slight movements of part of the skin of the head of the individual. This biomotion can be detected in a corresponding frequency range of the detection sensor signal.

For instance, the spatial sensor 7 can be configured to provide a detection sensor signal which a frequency portions related to biomotion referring to vibrations or periodic changes of external or internal boundaries of the head of the individual. These frequency portions can be related to respiratory activities and heartrate depending on their respective frequencies as described above. The detection sensor signal may be monitored correspondingly.

Furthermore, biomotion of an external or internal boundary can be caused by other body functions such as heart-beat, emission of sound by vocal cords and the like. The accord of the biomotions are detected and reflected in the detection sensor signal. By applying suitable filters 74 a heart-rate signal (signal portion in a range between 50 and 150 Hz) and a sound signal emitted by the individual (signal portion in a range above 150 Hz) can be detected.

An upcoming sleep apnea event can be determined by monitoring the detection sensor signal which has characteristics of a low frequency of 0,5Hz to 2Hz during normal respiration. The low frequency portion of the detection sensor signal is compared with a predetermined respiratory pattern and in case of a resemblance between the low frequency portion of the detection sensor signal and the predetermined respiratory pattern an upcoming sleep apnea event may be detected.

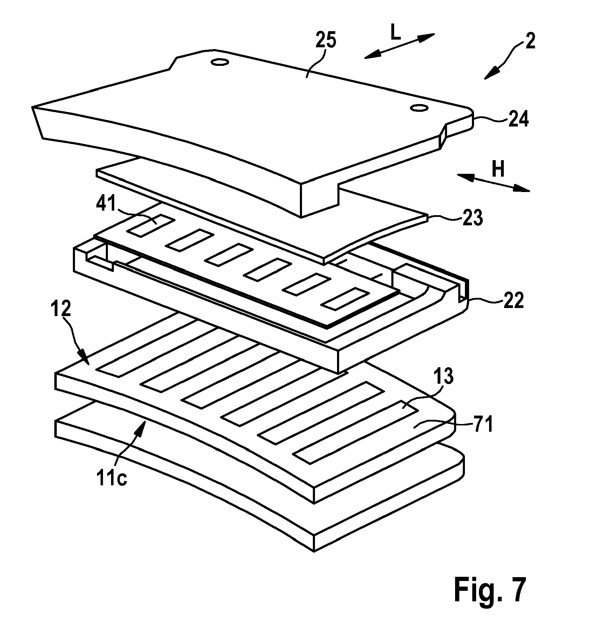

Figure 7 shows an exploded view of a further head support 2 with a capacitive detection means as the detection means 71 of a spatial sensor 7.

As in the embodiments described above, the head support 2 comprises a number of layers which includes a base layer 21 providing a rigid base support and the active layer 4 for positioning the deformation elements 41 and a frame means 22 for keeping it in a predefined position. On top of the active layer 4, it may be provided a buffer layer 23 for (damping) flattening the surface contour and a cover layer 24 providing the upper surface 25 on which the head of the individual resting thereon rests.

The capacitive detecting means 71 may have an electrode arrangement 12 of a plurality of electrodes 13 forming a substantially plane array. The electrodes 13 are electrically insulated with respect to each other. The electrode arrangement 12 may be formed with a number of electrodes 13 each having a same size and which are aligned in a row with an equal distance thereof. In other embodiments different sizes or differing distances are appropriate as well. The electrodes 13 may have a rectangular or irregular shape and their alignment may be along the lateral direction H so that a lateral position of the head of the sleeping individual can be detected.

The detection of the position of the head or of a biomotion is performed by measuring a capacity or respectively a capacity change of one or more of the electrodes 13 and a ground/reference potential in the signal extraction means 75. Since the capacity of each of the electrodes 13 is substantially influenced by electrically conducting body parts a detection of a position of the head or other body parts or biomotions thereof is possible.

Particularly, the proximity of a body part will result in an increase of measured capacity. Therefore, the head position is correlated to the capacitances measured at each of the electrodes 13. Is the capacitance of one or more neighbored electrodes 13 increased (exceeding a predetermined threshold) the presence of the head can be assumed at the lateral position of the respective electrodes 13.

For detecting the position of a head, the electrode 13 which has the highest capacitance to ground or to any other of the electrodes as a reference can be identified as the electrode above which the head is located. Detection accuracy/resolution can be improved by interpolating the measured capacitances or increasing the number of neighbored electrodes.

The electrode arrangement 12 of the capacitive detection means 71 may be located under (as shown) or above the active layer 4 as long as the distance between the head and the electrode arrangement 12 is within a sensing R, R' range of the electrical field detecting means. For instance, the electrodes may be deposited as a coating on the deforming elements 41.

Substantially, the electrodes 13 of the capacitive detection means 71 may have an extension along the different positions of the head on the head support 2 to be detected and can be arranged in/on any portion of the head support 2 as long as detection of movements of the head on the head support 2 is not affected. The electrode arrangement 12 of the capacitive detection means 71 may substantially extend along the deforming elements 41 in at least one lateral direction thereof. An information about the lateral position of the head is important for identifying the deforming element(s) 41 which has/have to be actuated to move the head. So the number of neighbored electrodes 13 of electrode arrangement 12 is selected depending on the accuracy for position detection required for identifying the deforming elements 41 to be actuated for moving the head.

If the capacitive detection means 71 is configured to have a sensing range R substantially directed in the upward direction, both the position of the head of the sleeping individual resting on the upper surface of the head support 2 and a biomotion of an external and inner boundary of the head, neck or chest of the sleeping individual can be detected. Particularly, the biomotion of an external or inner boundary of the head of the sleeping individual can be detected independent of the position of the head resting on the head support 2.

The control means 62 is configured to drive the deformation elements 41 in case an ongoing and/or upcoming airways disorder event is detected. The deformation elements 41 are driven so that a head resting on the head support 4 will start being moved thereby preventing the sleep apnea event to occur or causing the sleeping individual to start breathing again.

Particularly, the control unit 62 and/or the capacitive detection means 71 may be configured to detect periodic variations of the resulting measured capacities which may relate to low frequency conductivity changes of the skin of the sleeping individual due to blood flow and or slight movements of part of the skin of the head of the individual due to breathing activities. This biomotion can be detected in a corresponding frequency range of the detection sensor signal.

## Claims

1. Airways disorder alleviating system (1) for stopping an ongoing and/or upcoming airways disorder of a sleeping individual, comprising:
- a resting surface (3) on which the individual at least partly is to rest;
- a head support (2) with an active layer (4) being configured to selectively adapt the height of the head support (2) section by section;
- at least one spatial sensor (7) with at least one detection means (71) arranged in or on at least one of the resting surface (3) and the head support (2) for contactlessly detecting at least one of a position of a body part and a biomotion of a body part in a sensing range (R, R'), wherein particularly the at least one spatial sensor (7) is configured to use measurable field distortions of electromagnetic, sonic or electrical fields for contactless detection; and
- a control unit (6) configured to actuate the active layer (4) for selectively adapting the height of the head support (2) depending on the at least one of the detected position and the detected biomotion so that the head of the individual is moved.

2. System (1) according to claim 1, wherein the at least one spatial sensor (7) further comprises a signal extraction means (75) for receiving a detection sensor signal from the detection means (71) and for extracting at least one of a position sensor signal indicating the position of the body part of the sleeping individual and a biomotion sensor signal indicating periodic body characteristics of the sleeping individual from the at least one detection sensor signal.

3. System (1) according to claim 2, wherein the detections means (71) is configured to have a sensing range (R, R') which is directed one of upward perpendicular to the resting surface and upward inclined in a plane defined by the direction perpendicular to the resting surface and by a longitudinal direction of the resting surface (3).

4. System according to claim 2 or 3, wherein the control unit (6) is configured to detect an upcoming and/or ongoing airways disorder, particularly a sleep apnea event, depending on the at least one biomotion sensor signal indicating a respiratory activity of the sleeping individual as a body characteristics.

5. System according to claim 4, wherein the body characteristics include at least one of a blood-oxygen saturation, a heart-rate, a body movement pattern, vibrations, and a sound emitted by the individual, wherein the control unit (6) is configured to determine an upcoming and/or ongoing sleep apnea event if at least one of the body characteristics is paused for a predetermined time and a predetermined body characteristics pattern is detected.

6. System according to any of the claims 1 to 5, wherein at least one detection means (71) of the at least one spatial sensor (7) is arranged one of in and on the resting surface (3) and is displaced from the head support (2) along a longitudinal direction of the resting surface (3).

7. System according to any of the claims 1 to 6, wherein at least one detection means (71) of the at least one spatial sensor (7) is arranged at least one of on and in the interior of the head support (2).

8. System according to any of the claims 1 to 7, wherein the signal extraction means (75) of the at least one spatial sensor (7) is arranged in one of the head support (2) and the control unit (6).

9. System according to any of the claims 1 to 8, wherein the at least one detection means (71) includes one of:
- an antenna, wherein the at least one spatial sensor (7) is configured to emit radio frequency energy via the antenna and to provide the at least one detection sensor signal indicating the respiratory activity particularly by using a Doppler effect; and
- a capacitive proximity detection means, wherein the at least one detection means (71) includes an electrode arrangement (11) having a number of electrodes (11 a, 11 b, 11 c), wherein the detection means (71) is configured to generate an electrical field in the sensing range (R, R'), wherein the at least one spatial sensor (7) is configured to detect a distortion of the electrical field due to at least one of a position and a biomotion of a body part of the sleeping individual and to provide at least one detection sensor signal indicating at least one of the position of the body part and the respiratory activity.

10. System according to any of the claims 1 to 8, wherein the detection means (71) includes a capacitive detection means, wherein the at least one detection means (71) includes an electrode arrangement having a number of electrodes which are particularly neighbored and substantially arranged along a direction, wherein the signal extraction means (75) is configured for measuring at least one capacity between at least two of the electrodes and for providing at least one detection sensor signal depending on the at least one measured capacity so that at least one of a position sensor signal indicating the position of the body part of the sleeping individual and a biomotion sensor signal indicating periodic body characteristics of the sleeping individual is extractable from the at least one detection sensor signal.

11. Head support (2) for stopping an ongoing and/or upcoming airways disorder of a sleeping individual, comprising:
- an active layer (4) being configured to selectively adapt the height of a upper surface the head support (2) in sections;
- at least one detection means (71) of at least one spatial sensor (7) arranged on the upper surface of the head support (2) or in the interior of the head support (2) for contactlessly detecting at least one of a position of a body part and a biomotion of a body part in a sensing range (R, R'), wherein particularly the at least one spatial sensor (7) is configured to use measurable field distortions of electromagnetic, sonic or electrical fields for contactless detection; and
- a control unit (6) configured to actuate the active layer (4) for selectively adapting the height of the head support (2) in sections depending on the at least one detected position and detected biomotion so that the head of the individual is moved.

12. Head support (2) according to claim 11, wherein the detection means (71) is configured to have the sensing range (R, R') directed one of upward perpendicular to the upper surface and upward inclined in a plane defined by the direction perpendicular to the upper surface and by a longitudinal direction of the resting surface (3).

13. Head support according to claim 11 or 12, wherein the at least one detection means (71) includes one of:
- an antenna, wherein the at least one spatial sensor (7) is configured to emit radio frequency energy via the antenna and to provide the at least one detection sensor signal indicating the respiratory activity using a Doppler effect; and
- a capacitive proximity detection means with an electrode arrangement having a number of electrodes, wherein the detection means is configured to generate an electrical field in the sensing range, wherein the at least one spatial sensor is configured to detect an distortion of the electrical field due to at least one of a position and a biomotion of a body part of the sleeping individual and to provide the at least one detection sensor signal.

14. Head support according to claim 11 or 12, wherein the at least one detection means (71) includes a capacitive detection means, wherein the at least one detection means (71) includes an electrode arrangement having a number of electrodes which are particularly neighbored and substantially arranged along a direction, wherein the signal extraction means (75) is configured for measuring at least one capacity between at least two of the electrodes and for providing at least one detection sensor signal depending on the at least one measured capacity so that at least one of a position sensor signal indicating the position of the body part of the sleeping individual and a biomotion sensor signal indicating periodic body characteristics of the sleeping individual is extractable from the at least one detection sensor signal.

15. Head support according to any of the claims 11 to 14, wherein the detection means is arranged one of below and above the active layer (4), wherein particularly the electrode arrangement has two electrode layers each with one or more electrodes wherein at least one of the electrode layers is formed on the deformation elements, particularly as a coated layer.
